# EUROPEAN PATENT APPLICATION

(11) **EP 1 505 395 A1**
(43) Date of publication of application: **09.02.2005**
(21) Application number: 03077471.5
(22) Date of filing: 07.08.2003
(51) Int. Cl.: G01N 33/94

(54) **Method for identifying active and/or inactive metobolites; and novel active metabolites**

(71) Applicant: Kiadis B.V., 2333 CA Leiden (NL)
(72) Inventor: Irth, Hubertus, 1078 NP Amsterdam (NL); Schenk, Timothy, 1073 BB Amsterdam (NL)
(74) Representative: Prins, Adrianus Willem, Mr. Ir.

(57) **Abstract**

The present invention relates to a method for determining whether or not a metabolite of a particular lead compound is an active or an inactive compound. Particularly, this method allows an early screening on whether or not a lead compound itself or an active metabolite thereof is a suitable candidate to go through the time- and money consuming route to try and obtain a valuable and marketable pharmaceutical composition. In a second, aspect, the present invention relates to active metabolites found by carrying out the method of the invention.

## Description

The present invention relates to a method for identifying active and/or inactive metabolites. It is intended to provide a profiling method that, within a limited time frame, allows to determine for a number, and generally a large number, of metabolites (or other types of conversion products, which other types of conversion products are also intended to be covered by the term "metabolites") whether these are active or not active in a particular interaction with a biomolecule or other pharmaceutically interesting target. Further, the invention relates to new active compounds which are found in the method of the present invention. The invention is particularly useful in the area of drug discovery and allows the early profiling and ranking of so-called "lead compounds".

Over the years, many technologies have been developed that facilitate and accelerate the discovery of novel chemical entities (NCEs). Such NCEs can, for instance, after passing various stages of clinical trials, be developed into a new pharmaceutical product. More and more, the development of NCEs starts from large libraries of chemical components. Such libraries can be prepared by combinatorial synthesis or derived from natural products or other complex mixtures.

Very suitable methods to identify NCEs that are potentially promising candidates for pharmaceutical compositions, and can be suitable starting compounds for the method of the present invention are described in the following publications: U.S. Patent No. 6,080,590; EP-A-0 821 790; and the international patent applications WO-A-00/65354, WO-A-00/65353 and PCT/NL03/00408. These references are incorporated by reference to describe methods for finding "lead compounds" or "active components" useful as starting compound in the method of the present invention.

In most primary screening programs, High-Throughput Screening techniques are used to identify so-called hit molecules. These hit molecules are selected based on their affinity for particular biomolecular or other pharmaceutically or diagnostically interesting targets such as receptors and enzymes.

The measurement of the interaction of chemical components with these targets forms a useful step in drug discovery, and large efforts are made to develop and validate biochemical assays for this purpose.

A bottleneck in drug discovery is the characterization of hit molecules with respect to their "drug-like" properties. In this light, reference is made to an overview of Caldwell in Current Opinion in Drug Discovery & Development 3(1) (2000), 30-41. Caldwell notes that the goal of what he calls the "hit generation" step is to screen large compound libraries in a relatively short amount of time in an attempt to find compounds that cause a specific biological response, *"hits".* This step includes target identification, selection, validation and high-throughput screening (HTS) of large structurally-diverse compound libraries. The productivity in target identification and selection has improved with the development of automated DNA sequencing, genomics databases and bioinformatic tools. However, target validation remains a time-consuming process where assays are typically performed without automation. HTS operations are highly automated to handle sample preparation, assay procedures and large volume data processing. After each step is optimized to operate efficiently, it is common to screen 100,000 compounds in a 1- to 6-month period. This characterization process will end with so-called lead molecules that subsequently enter clinical trials.

Many NCEs have to be abandoned during this process since they appear to be toxic, have unfavorable absorption properties or metabolic profiles. It is of importance for an efficient and economic drug discovery process to de-select all those compounds that exhibit unfavorable pharmacokinetic properties at the earliest possible stage. For this purpose, a large variety of *in vitro* tests are developed and used to characterize lead compounds prior to clinical trials. These tests are commonly referred to as "early ADME".

One of the key parameters to be determined in early ADME is metabolic stability of novel drug candidates. Metabolism refers to the elimination of chemical compounds foreign to the human or animal body after biotransformation. The majority of biotransformation reactions is promoted by the cytochrome P450 enzyme system which is mainly located in the liver, and this cytochrome P450 enzyme system is also a preferred metabolite generating system used in the method of the present invention.

Metabolites formed in this process are typically more hydrophilic and hence easier to eliminate. Although most often biotransformation results in inactivation of the drug, some compounds form active metabolites. These active metabolites can enhance, modify or inhibit the desirable activity of the drug. In some cases metabolites may exhibit toxic effects. It is therefore important to understand the number and type of metabolites derived from candidate drug molecules as early as possible. Knowledge of the metabolic pathways, metabolite stability, toxicity and the specific isoenzymes involved in the metabolism are all important information in the NCE selection process.

The present invention aims to provide methods which have benefits over the known metabolic profiling methods. Such known methods for early stage metabolic profiling can principally be divided into two categories, the computational methods ('*in silico*'), and the *in vitro* methods.

Computational methods make use of molecular modeling principles in combination with the 3D structure of metabolic enzymes to determine whether a particular lead compound may serve as substrate or inhibitor for specific isoenzymes. Computational methods are generally used in addition to *in vitro* methods.

*In vitro* methods involve the incubation of test compounds with preparations containing metabolic enzymes. These preparations may include purified enzymes (for example, CYP2A6) produced by recombinant DNA methods, microsomes (prepared from liver tissues) containing most of the oxidative drug metabolising enzymes, isolated hepatocytes (primary cells from fresh liver tissue) or tissue slices from liver tissue containing the complete complement of drug metabolizing enzymes. The enzyme (containing) systems mentioned are also useful in the method of the present invention.

*In vitro* methods may be used to give information on metabolic stability (i.e., half-lifes of parent compounds and metabolites), metabolic profile (i.e., the number and abundance of individual metabolites), metabolite identity, interspecies comparison, drug-drug interaction studies, CYP isoform identification and phase II enzyme studies.

Of particular interest is the distribution between active and non-active metabolites. Active metabolites exhibit a similar, sometimes even stronger pharmacological behavior as the parent active compound or lead compound. In some cases it turned out that an active metabolite of a lead compound is preferable as drug candidate. On the other hand, an active metabolite may also exhibit a toxic behavior rendering the parent active compound useless as potential drug. Similar to the parent lead compound, active metabolites show affinity for the biomolecular target associated with the drug discovery program. Active metabolites therefore can preferably be detected with the same high-through screening assays as the parent lead compound.

Recently, several methods have been described for studying *in vitro* metabolism of NCEs. In most cases, the methods to determine the chemical profile of metabolites (measurement of concentration, identification of metabolites) are significantly different from the methods to determine the biological profile, and, in particular, to determine whether a metabolite is active towards the biomolecular target used for screening.

Samples for metabolic profiling may contain the following components,
the parent lead compound, or "active component";
non-active metabolites;
active metabolites;
and, in addition, components from the preparation used to metabolise the active component, generally by incubation of the active component, *e.g.* membrane-bound enzymes (cytochrome P450 enzyme system) and soluble proteins, buffer ingredients, and co-factors, will be present.

Characteristic to all metabolic profiling experiments is the fact that parent compound and metabolites are present in more or less complex mixtures of chemical components. Furthermore, there may be considerable concentration differences between the parent compound and minor or major metabolites formed during the incubation experiment. Any method for metabolic profiling providing the information mentioned above therefore has to be capable to measure the desired parameters in mixtures of compounds rather than in samples containing only a single component.

The determination of the total metabolic profile is nowadays generally performed using analytical techniques based on liquid chromatography-mass spectrometry (LC-MS). A large number of documents have been published on the determination of parent compounds and their metabolites in samples deriving from *in vitro* or *in vivo* experiments. Typically, a sample pretreatment step is carried out to remove interfering sample constituents such as proteins and other biomacromolecules. Subsequently, the sample is analyzed by LC-MS.

Tiller *et al*. in Rapid Commun. Mass spectrom. 16 (2002), 1225-1231 give an overview on the use of LC-MS in the early drug discovery process. Throughput for early discovery drug metabolism studies can be increased with the concomitant acquisition of metabolite screening information and quantitative analysis using ultra- fast gradient chromatographic methods. Typical ultra-fast high-performance liquid chromatography (HPLC) parameters used during early discovery pharmacokinetic (PK) studies, for example, employ full-linear gradients over 1-2 min at very high flow rates (1.5-2 mL/min) on very short HPLC columns (2 x 20 mm). These conditions increase sample throughput by reducing analytical run time without sacrificing chromatographic integrity and may be used to analyze samples generated from a variety of *in vitro* and *in vivo* studies. They emphasize that this approach allows acquisition of more information about a lead candidate while maintaining rapid analytical turn-around time.

A further example of state-of-the-art for the determination of metabolic profiles is presented by Jones *et al*. who studied the metabolism of tamoxifen. In Rapid Commun. Mass Spectrom 13 (1999), 211-215, Jones *et al*. describe an on-line high-performance liquid chromatographic (HPLC)-electrospray ionization mass spectrometric (ESI-MS) to study metabolism in mouse liver microsomes. Mouse liver microsomal preparations were incubated with tamoxifen in the presence of NADPH and MgCl₂. The metabolites formed were separated and analyzed by HPLC-ESI-MS. The separation was performed on a Res Elute-BD column (5 µm particle size, 250 x 4.6 mm LD) with 70% (v/v) methanol in 0.5 M ammonium acetate as the mobile phase. A total of eleven metabolites were detected. The metabolites identified are: tamoxifen N-oxide, N-desmethyltamoxifen, 4-hydroxytamoxifen, 4'-hydroxytamoxifen, 4-hydroxytamoxifen N-oxide, 4'-hydroxytamoxifen N-oxide, 4-hydroxy-N-desmethyltamoxifen, 4'-hydroxy-N-desmethyltamoxifen, 3,4-dihydroxytamoxifen, 3,4-epoxytamoxifen and 3,4-epoxytamoxifen N-oxide.

Lim *et al*. present in J. Chromatogr. A 831 (1999), 227-241, a rapid drug metabolite profiling method using fast chromatographic separation and fast mass spectrometric scanning. They show how fast chromatographic separations of a particular drug and its metabolites can be achieved by eluting from a short narrow-bore guard cartridge column (20x2 mm I.D., 3 µm BDS Hypersil C-8) at a flow-rate of 1.0 ml/min and with a gradient volume greater than 90 column volumes. The total analysis time of 8 min permits profiling of metabolites in a 96-well plate in 13 h. The narrow chromatographic peaks resulting from the high flow-rate require the use of a mass spectrometer capable of fast scan speed due to the need to perform multiple MS experiments within the same chromatographic analysis.

The methods mentioned in the previous three paragraphs provide information on the total number of metabolites present in a sample. These methods also allow the measurement of the time course of metabolites formation. With the LC-MS methods summarized above, however, it cannot be determined whether a certain metabolite is active or not. Methods for the determination of metabolite activity are often based on biochemical assays carried out in microtiter plates. For this purpose, the individual metabolites have to be isolated from the metabolic preparation, and subsequently purified by HPLC. The purified, isolated metabolite is then subjected to the biochemical assay, which is based on the interaction with the biomolecular target of the drug candidate.

An example for the identification and characterization of active metabolites is presented by Pereillo *et al*. in DMD 30 (2002) 1288-1295. They describe the metabolic activation of a drug called clopidogrel in the human liver resulting in the formation of active metabolites. In order to identify active metabolites of clopidogrel, the compound was incubated with human liver microsomes. Subsequently, a first purification step was carried out by semipreparative liquid chromatography. Various fractions were collected and subsequently freeze-dried. A second preparative purification step using semipreparative HPLC was carried out to separate and isolate the individual metabolites. Finally, the activity of the isolated fractions was tested in an in-vitro bioassay by measuring the *in vitro* activity of the metabolites on the binding of 33P-2MeS-ADP human platelets.

A similar approach is described in Clinical Chemistry 45 (1999), 419-422 by Schütz *et al*.. They present a method allowing the identification of a pharmacologically active metabolite of mycophenolic acid in plasma. For this purpose they used an HPLC fractionation method to isolate and purify the metabolites by HPLC. After collection, the fractions containing metabolites were desalted by column chromatography, concentrated by evaporation and reconstituted in water. The purity of the isolates was then confirmed by rechromatography on HPLC. The activity of the isolates compounds was subsequently tested in a biochemical assay.

In summary, the known methods for the determination of active metabolites currently used comprise the following steps:
(1) incubation of the test compound with a metabolic preparation;
(2) sample pretreatment for the removal of interfering constituents;
(3) fractionation of the sample by (semi)preparative HPLC;
(4) isolation and further purification of the metabolites;
(5) evaporation/removal of HPLC solvents; and
(6) testing of the individual metabolite fractions in an *in vitro* bioassay.

Steps 1 to 5 are carried out on chemical analysis instruments while step 6 is normally carried out on a screening instrument.

Due to the need of repeated fraction collection and the transfer of fractions from analytical instruments to screening instruments, the way of determination of active metabolites known in the state of the art is very cumbersome and time-consuming. The complicated logistics of fraction isolation, treatment and transfer to screening instruments becomes particularly costly and elaborative in the area of early ADME, *i*.*e*., in *in vitro* screening of lead molecules. The number of compounds to be tested in early ADME is typically by a factor of 10 or more higher than in preclinical metabolic studies. Methods that would circumvent the sample transfer between screening and chemical analysis instruments would therefore be very valuable and provide a high throughput and quality of information.

A possible solution to avoid the transfer of samples between screening and chemical analysis instruments is the use of LC-MS for the detection of both active and non-active metabolites. Van Breemen *et al*. developed a pulsed ultrafiltration-mass spectrometric screening method to evaluate the metabolic stability of drugs. (See: Drugs Metabolism and Disposition 26 (1998), 85-90). Pooled human liver microsomes containing cytochrome P450 enzymes were trapped by an ultrafiltration membrane in a stirred flow-through chamber, and 8 beta-blocker drugs including acebutolol, alprenolol, atenolol, metoprolol, oxprenolol, pindolol, propranolol, and timolol were flow-injected through the chamber along with the cofactor NADPH. The ultrafiltrate was collected, concentrated and analyzed by using liquid chromatography-tandem mass spectrometry (LC-MS-MS) in order to quantify the unmetabolized fraction of each drug. The metabolic stability of each beta-blocker was determined based on the difference between the corresponding LC-MS-MS peak areas of an experimental incubation and a control without NADPH.

In their above-identified article, Lim *et al*. proposed a method where mass spectrometry is used to determine whether a metabolite is active towards a certain molecular target. They have developed a method for screening potentially biologically active *in vitro* microsomal metabolites by affinity binding with a receptor. After separation by centrifugal ultrafiltration, the bound ligands are released and characterized by LC-MS. As an example, the determination of active metabolites of tamoxifen is described. The *in vitro* metabolites of tamoxifen captured by the receptor include N-demethyltamoxifen and three species of hydroxytamoxifen; these data are consistent with those from a conventional binding study and bioassay. In addition, both hydroxyraloxifene and dihydroxyraloxifene are also recognized by the receptor. The specificity of the molecular recognition process was illustrated by the absence of binding with control microsomal incubate and with adatanserin and its metabolites.

While the methods proposed by Lim *et al*. and Van Breemen *et al*. are capable to identify active metabolites by measuring the interaction with a receptor, still two different methods are required to identify both the total (active and non-active) and active metabolites. Moreover, the incubation methods described by Lim *et al*. and Van Breemen *et al*. have a serious disadvantage in that low concentrations of active metabolites are potentially displaced from the receptor by the relatively high concentration of the lead compound used for the incubation experiment. Typically, the lead compound is selected based on its affinity for the receptor. The metabolic sample may, depending on the time of incubation, still contain a significant concentration of the parent lead compound next to the metabolites formed. If the receptor is incubated with the metabolic samples without preseparation of the parent compound and the metabolites, low concentrations of active metabolites may not be detected, particularly if low receptor concentrations are used for the screening measurement.

The present invention seeks to overcome or at least to reduce the problems associated with both the fractionation method (Pereillo *et al*.) and the receptor incubation method (Lim *et al*. and van Breemen *et al*.). A method is proposed wherein active and non-active metabolites are simultaneously characterized in *in vitro* metabolic profiling samples. The same method can also be used for the simultaneous characterization of active and non-active metabolites in *in vivo* samples such as human plasma.

More particularly, the present invention is based on chemical analysis systems that allow the simultaneous measurement of chemical and biochemical characteristics of metabolites that are present in a mixture derived from a metabolic profiling experiment.

In a first aspect, the present invention relates to a method for identifying active and/or inactive metabolites of at least one active component, comprising the steps of:
a) providing a mixture starting from the at least one active component, by subjecting said at least one active component to a metabolising step giving active and/or inactive metabolites;
b) fractionating said mixture;
c) dividing the fractionated mixture in at least two fractionation streams;
d) subjecting one of the fractionation streams to a biochemical detection;
e) subjecting in parallel another fractionating stream to a chemical characterization step;
f) determining on the basis of the parallel results provided by steps d) and e) whether the metabolites present are active.

In the context of the present invention, chemical characteristics of a metabolite are *e.g.* its UV-VIS absorption spectrum, its mass spectrum and its fragmentation spectrum obtained after MS-MS experiments. Furthermore, information on the concentration of the metabolite is considered.

In the biochemical detection step the ability of the metabolite to bind to a biomacromolecule such as a receptor or an enzyme that is used to the screen for drug candidates to be used in certain disease areas is determined. The binding of the metabolite to such a biomacromolecule indicates that the metabolite may be biologically active, *i.e.*, exhibit the same pharmacological properties as the parent lead compound.

Preferred fractionation steps generally are liquid chromatography steps and comprise HPLC, reversed phase HPLC, capillary electrophoresis (CE), capillary electrochromatography (CEC), isoelectric focusing (IEF) or micellar electrokinetic chromatography (MEKC), all of which techniques are known to the person skilled in the art. In a preferred embodiment, the liquid chromatography separation step is a reversed phase HPLC step.

The method of the present invention also enables the screening of metabolites in mixtures. "Mixtures" in the present description refer to samples derived from *in vitro* or *in vivo* metabolic experiments. *In vitro* metabolic experiments may provide mixtures that contain, next to the parent compound, an unknown number of metabolites as well as components of the metabolic preparation (microsomes, hepatocytes, isolated enzymes) which is used in the metabolic experiment. Samples derived from in vivo metabolic experiments are considerably more complex and may contain a large number of compounds ranging from low to high molecular mass constituents.

The present invention also encompasses analytical methodologies that simultaneously perform chemical analysis and biochemical screening of the metabolic samples.

The analytical system of the present invention proposed comprises three main elements, *i.e*., a separation method capable to separate the parent lead compound and the metabolites formed during incubation with a metabolic preparation, an on-line continuous-flow biochemical detection system for the biospecific detection of active metabolites and an on-line mass spectrometric detection system for the detection of all metabolites (both active and non-active metabolites).

This makes that the method of the present invention allows to determine at least the following parameters:
- the possibility of metabolising using a particular metabolising system;
- time-dependent measurement of the concentration of the active starting compound and metabolites formed during an incubation period;
- chemical identification of all metabolites formed during the incubation period;
- identification of active metabolites, i.e. those metabolites that interact with the biomolecular target, *e.g.* a particular enzyme or receptor, used in the biochemical detection step; and
- determination of inhibition of metabolic enzymes.

In comparison to the methods described by Lim *et al*. and Van Breemen *et al*., the present invention provides several advantages.

First and most importantly, weak affinity ligands or active ligands present at low concentrations are more likely to be detected with the present invention since incubation with the receptors takes place after the different components are separated by for instance HPLC.

Secondly, since the receptor-ligand interaction takes place downstream to the fractionation, it is possible to implement trace enrichment methodologies prior to for instance the LC separation (for example, using solid-phase extraction), thereby significantly lowering the detection limits of the screening method; the method reported by Lim *et al*. would require the use of higher amounts of receptor for this purpose, significantly increasing the costs of the method.

Thirdly, the screening method of the present invention is not affected by different incubation conditions, for example, different metabolic preparations, since the receptor ligand interactions are placed downstream to the separation step in preferably an LC column; the method presented by Lim *et al*. is used prior to LC separation and MS detection and is therefore strongly influenced by different incubation conditions.

In comparison to the LC-fractionation approach described by several authors *(vide supra)* the present method provides at least the following advantages:

Screening and chemical analysis of metabolites are carried out in one measurement, therefore the tedious fraction collection and transport of samples between screening instruments and chemical instruments using the LC-fractionation approach is avoided.

Further, closely overlapping peaks of metabolites can be detected by mass spectrometry and reanalyzed under gradient conditions adapted to the specific retention time of the ligand.

The present invention will be described in more detail while referring to the drawings wherein:
Figure 1 summarizes the configurations in which the said three essential elements are assembled;
Fig. 1A shows a configuration using HPLC, biochemical detection and mass spectrometry (MS);
Fig. 1B shows a configuration using HPLC, biochemical detection, native fluorescence-DAD-ELSD-MS;
Fig. 2 shows a continuous-flow biochemical assay for the screening of phosphodiesterase used in Example 1;
Fig. 3 shows the screening configuration and screening results obtained in example 1;
Fig. 4 shows a continuous-flow biochemical assay for the screening of the estrogen receptor used in Example 2;
Fig. 5 shows the screening configuration and screening results obtained in example 2; and
Fig. 6 shows the mass spectrum obtained for peak no. 1 of the bioactivity chromatogram (data trace A, Fig. 5).

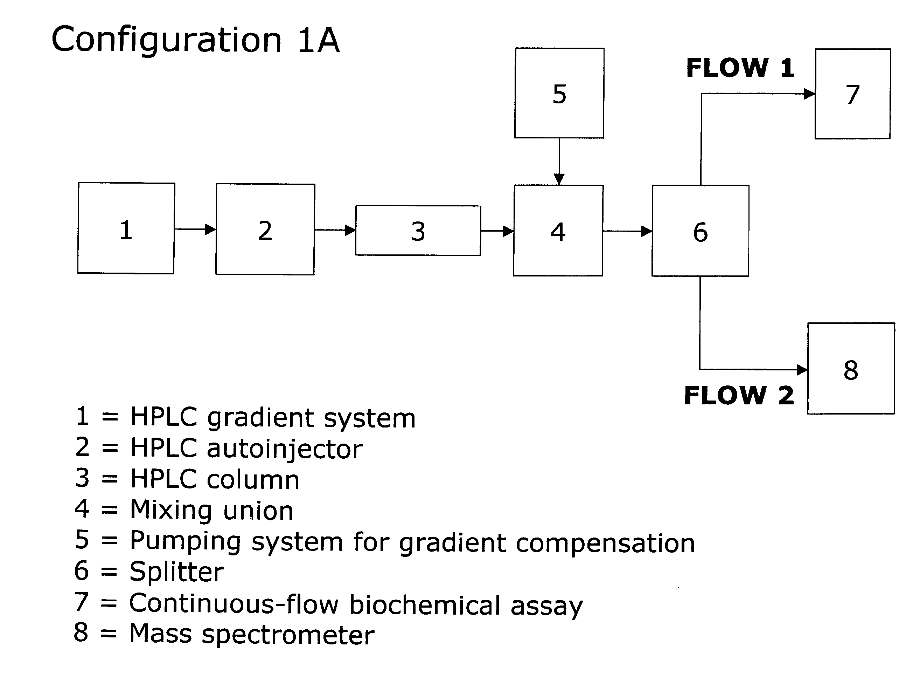

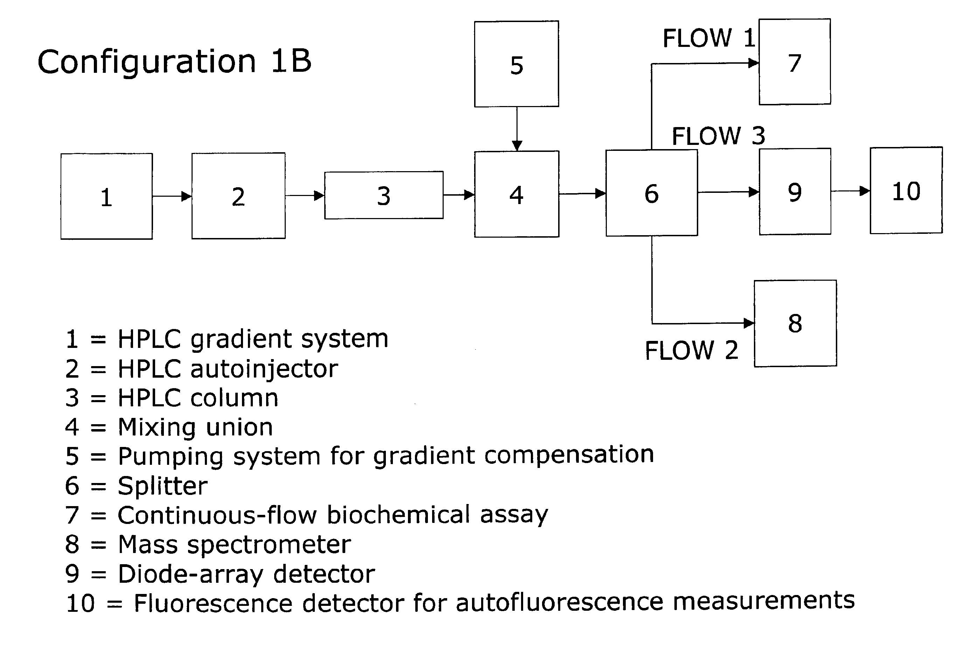

Figure 1A depicts a basic system used for simultaneous screening and chemical analysis. The system comprises an HPLC pump, an autoinjector 2, a mixing union 4 for modification of the HPLC eluate, and a flow splitter 6 that divides said HPLC eluate into two streams. One part (FLOW 1) is directed towards a continuous flow biochemical detection system 7 based on the receptor or enzyme used for bioactivity screening. The second part (FLOW 2) is directed towards a mass spectrometer 8 or any other chemical detector providing information on the chemical characteristics of metabolites.

The metabolic sample is injected by the autoinjector 2 into HPLC separation system 3. In case a gradient is used for elution the amount of organic modifier is adjusted to a constant value by the addition of water via a mixing union 4 downstream to the HPLC column 3. The metabolites separated by HPLC are split into two flow streams directed towards the continuous flow biochemical assay on the one hand (FLOW 1) and to the mass spectrometer on the other hand (FLOW 2). In the biochemical detection system (FLOW 1), each metabolite is allowed to interact with the receptor or enzyme used for bioactivity measurement. The interaction is preferably measured by using fluorescent reporter molecules in combination with fluorescence detection. Active metabolites give a measurable response in the biochemical assay while non-active metabolites are not detected at all. Metabolites directed towards the MS (FLOW 2) are detected independently on their activity.

Peaks obtained in the biochemical detection line can be correlated with peaks in the mass spectrometric detection line. This is *e.g.* possible when both lines have a constant hold-up (dead) volume and both flow rates (FLOW 1 and FLOW 2) are kept constant throughout the chromatogram. Metabolites giving a response in the biochemical detection line are indicated as "active metabolites".

The molecular mass information measured for an active metabolite detected in the biochemical detection line is obtained from the total mass spectrum at the correlated retention time. Typically, the reconstructed ion chromatograms (RIC) of the most abundant m/z values are established, and the m/z with the smallest difference between the expected and measured retention time is selected as most likely m/z value belonging to the active metabolite. If the mass spectrometer was operated in the MS-MS mode, fragmentation spectra of the parent mass of the active metabolite are collected as well and used for further structure elucidation and identification.

In a preferred embodiment, quantitative information on the affinity of the active metabolite is obtained. For this purpose, a three-way split is installed allowing the addition of other chemical detectors (FLOW 3). The system is depicted in Figure 1B. In order to generate quantitative data on the affinity of the metabolite, it is necessary to measure its concentration. In the system of the present invention, an evaporative light scattering detector (ELSD) can be installed. An ELSD provides information of the concentration of a certain compound in the eluate of an HPLC column. In combination with the peak height in the biochemical detection line, an estimate on the IC₅₀ value can be obtained. This estimate becomes more accurate if the data for unknown compound are correlated with affinity data for a known active compound.

The UV or diode-array data obtained from FLOW 3 is used to facilitate structure elucidation. For example, a response in UV detection at a wavelength of 254 nm indicates the presence of benzene rings. This information can be used in structure generation program to limit the number of potential structures of the active metabolite.

In a further aspect, the present invention relates to the active metabolites that are found while using the method of the present invention. Further, the invention also encompasses the use of these new active metabolites in the manufacture of a medicament on the basis of its activity to the biomolecule, receptor, enzyme or any other suitable target used in the biochemical detection step of the method of the invention. Further details on the biochemical detection step are for instance described in the above identified U.S. Patent No. 6,080,590; EP-A-0 821 790; and the international patent applications WO-A-00/65354, WO-A-00/65353 and PCT/NL03/00408.

The invention will be further illustrated while referring to the following non-limiting examples.

### Example 1: Use of parallel chemical and biochemical detection

### Materials

Mant-Guanosine 3',5'-cyclic monophosphate (mant-cGMP) was purchased from Calbiochem (Darmstadt, Germany). Activator deficient phosphodiesterase 3', 5'-cyclic nucleotide (PDE), calmodulin, Hepes, bovine serum albumin (BSA), polyethylene glycol MW 600 (PEG), 3-isobutyl-1-methyl-xanthine (IBMX), theophylline and papaverine were obtained from Sigma-Aldrich (Steinheim, Germany). Dimethylsulfoxide (DMSO), methanol and acetonitrile were purchased from Baker (Deventer, the Netherlands). ELISA blocking reagent was obtained from Boehringer Mannheim. Natural products were kindly provided by Bayer (Wuppertal, Germany). All other chemicals were purchased from Merck (Darmstadt, Germany) and were analytical grade.

### Instrumentation

All experiments were carried out using a Gilson 235 auto-injector (Villiers-le-Bel, France) equipped with a Valco VICI ten-port injection valve (Schenkon, Switzerland), Agilent (Waldbronn, Germany) IsoPumps G1310A 1100 series, an MetaChem Technologies Inc. (Torrance, USA) MetaVac in-line 8-channel degasser and two Agilent fluorescence detectors 1100 series (excitation wavelength 280 nm, emission wavelength 460 nm) and an Agilent diode-array detector (DAD) detector 1100 Series (wavelength 254 nm). Two Shimadzu (Kyoto, Japan) TO-10ACvp column ovens were used to control the reaction temperature and the temperature of the LC separation. Two Pharmacia (Uppsala, Sweden) 50 ml Superloops placed in a Spark (Emmen, The Netherlands) column oven at 5°C were used for reagent addition using water as displacement solution. All tubing consisted of 0.13 mm i.d. PEEK, except for the reaction coil, which was made of 0.25 mm i.d. x 0.4 mm o.d. PTFE. In the HRS mode a custom made LC-Packings (Amsterdam, The Netherlands) flow-splitter was used. All instrument control and data acquisition, except for the MS, were performed using the Kiadis proprietary software program ScreenControl version 3.0 (Kiadis, The Netherlands). Data processing was done using the Kiadis proprietary software program ScreenAnalysis version 3.0 (Kiadis, The Netherlands).

Mass spectrometry was performed on a Micromass quadrupole-orthogonal-acceleration time-of-flight mass spectrometer (Q-TOF micro MS) equipped with a LockSpray (electrospray) source (Micromass Ltd., Wythenshawe, Manchester, UK). Instrument control, data acquisition and data processing were done using Masslynx software (version 4.0). The Q-TOF micro MS instrument was operated in 'full-scan' MS with a spectrum integration time of 1 s in the range of m/z 100-900 ('interscan' time 0.1 s). Acquisition was performed in continuum mode. The electrospray source conditions were: capillary voltage, 3.0 kV; sample cone voltage, 25 V; extraction voltage, 2 V; source temperature, 150°C; desolvation temperature, 350°C; cone gas, 50 l/h and desolvation gas, 600 l/h. Evaporative light scattering detection was performed using a Sedere (Allfort Ville, France) SEDEX 75 detector.

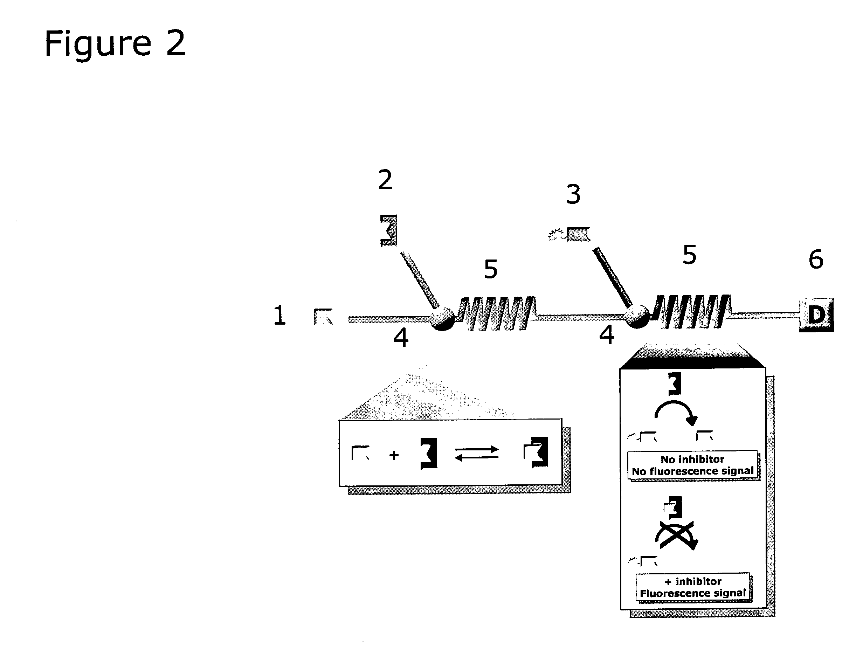

The principle of the on-line coupling of liquid chromatography to a detection system for the simultaneous monitoring of chemical and biochemical characteristics is outlined using an on-line phosphodiesterase system as example. The configuration of Fig. 1B has been used to generate chemical and biochemical information using the injection of a complex compound mixture containing two biologically active compounds, *i.e.,* theophylline and papaverine. The assay principle is depicted in Figure 2.

Briefly, the effluent of the HPLC column (FLOW 1) is mixed with a solution containing phosphodiesterase (PDE) allowing compounds eluting from the HPLC column to interact with the enzyme in the absence of substrate. A PDE specific substrate, Mant-cGMP, is added to the solution of reaction 1; a fluorescent moiety attached to the specific substrate is used to monitor this interaction by fluorescence detection. The fluorescence of Mant-cGMP is reduced upon its conversion by PDE to Mant-GMP. The binding of inhibitors to PDE results in an increase of the fluorescence signal since the production of Mant-GMP is temporarily reduced. The fluorescence of Mant-cGMP is detected by a standard HPLC fluorescence detector.

More in detail, a carrier solution flow consisted of 10 mM Hepes (pH 7.4), 90 mM KCl, 0.73 mM CaCl₂ and 5 mM MgCl₂ and was pumped at a flow rate of 100 µl/min. The PDE and mant-cGMP reagent solutions were kept at 5°C and were added to the carrier flow at 25 µl/min. PDE was dissolved in 10 mM Hepes buffer (pH 7.4) containing 90 mM KCl, 0.73 mM CaCl₂, 5 mM MgCl₂ and 4 U/ml calmodulin. Mant-cGMP was dissolved in 10 mM Hepes buffer (pH 7.4) containing 90 mM KCl, 0.73 mM CaCl₂ and 5 mM MgCl₂. The reaction coil had a volume of 150 µl. The influence of different parameters on the performance of the biochemical assay were studied and optimized, such as the PDE concentration, mant-cGMP, the reaction temperature. In addition, the ability to reduce non-specific binding of the assay reagents to the inner surface of the detection system was studied by dissolving different additives in the PDE solution. Finally, the influence of DMSO, methanol and acetonitrile on the assay performance were studied by adding increasing concentrations of these organic modifiers to the assay carrier using 10 µM mant-cGMP, 4 mU/ml PDE, a reaction temperature of 30°C and 0.05% BSA in the PDE solution.

The PDE is implemented into the screening system described by the present invention as shown in Figure 1B. The PDE is used to describe the data output provided by this screening system.

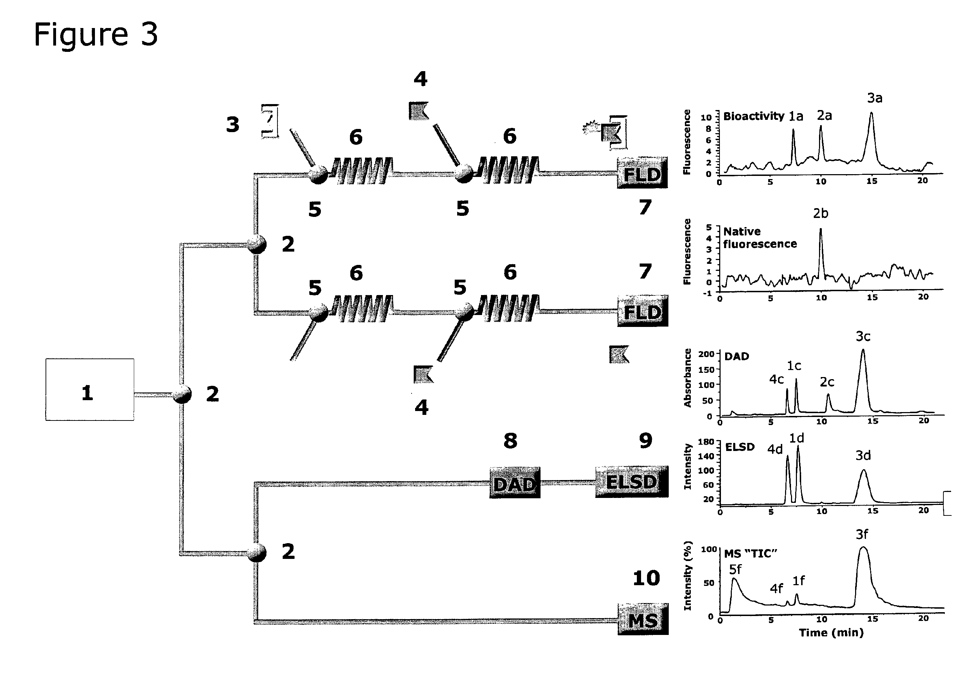

Figure 3 shows the total configuration and the chromatograms resulting from the screening of a complex mixture consisting of several unknown chemical components. The bioactivity profile shown in data trace (a) indicates that three bioactive compounds (1a, 2a and 3a) may be present in the mixture. Data trace (b) representing a native fluorescence measurement indicates that compound 2 (2b) is probably not biologically active but exhibits autofluorescence. Data trace (c) shows the UV properties of the compound mixture measured at 254 nm indicating that all three compounds possess UV absorptivity at this wavelength and, therefore, may contain phenolic rings. Compound 4 possesses UV absorptivity but is not biologically active. Data trace (d) reflects the response of an evaporative light scattering detector (ELSD); from the peak heights the approximate concentration of the different compounds can be determined. Data trace (f) represent the total ion current chromatogram detected by electrospray mass spectrometry. At the retention times corresponding to the retention times of the biologically active peaks, further MS data such as MS-MS data can be derived. From the MS data important information on the structure of the active compound can be obtained. Furthermore, database searches in MS libraries allow the user to establish whether the compound was already found in earlier screens.

More in detail, for the on-line coupling of the PDE assay to LC a Phenomenex (Torrance, USA) 50 x 2.0 mm i.d. stainless-steel column packed with Luna C18 3-µm particles and a 4.0 x 2.0 mm i.d. guard column packed with C18 5-µm particles were used. Separation of the compounds in the plant extract (injection volume, 50 µl) was performed with a linear gradient elution from 5 to 95 % methanol in 10 minutes, and held constant at 95% for an additional 5 minutes. A post-column splitter was inserted to obtain a continuous flow of 100 µl/min to both assay lines, 100 µl/min to the MS and 700 µl/min to the DAD and to the ELSD. The signals from the biochemical detection were synchronized with the signals from the MS and DAD/ELSD using a hold-up volume consisting of 0.5 mm i.d. PTFE tubing. 20 ng/µl erythromycin A dissolved in 0.1 % acetic acid/methanol (50/50%, v/v) was continuously added at 4 µl/min using the LockSpray. After data acquisition, lock mass adjustment was performed using erythromycin A (m/z 734.4690 due to the protonated molecule) to allow accurate mass determination.

Plant extracts in DMSO were centrifuged and the supernatant was diluted with water to a final DMSO concentration of 5%. Spiking of the plant extract was performed by adding an appropriate amount of theophylline and papaverine to a plant extract to obtain a final inhibitor concentration of 250 µM.

### Example 2: Screening of active metabolites of tamoxifen

β-Nicotinamide Adenine Dinucleotide Phosphate tetra sodium salt (NADPH) was from Applichem. Methanol (MeOH) was obtained from Riedel-de Haën. Acetonitrile was purchased from Baker. Both the MeOH and ACN were of HPLC reagent grade. All other chemicals were of the highest grade commercially available. Rat liver microsomes were prepared as described elsewhere (5). Protein concentration in the microsomes was 13.1 mg/ml. Pig liver microsomes were prepared the same way as the rat liver microsomes (livers of three pigs were used). Protein concentration in this microsomal preparation was 26,2 mg/ml.

The use of the present invention is described using the metabolic profiling of tamoxifen as example. The α-human estrogen receptoris used as the biomolecular target to screen tamoxifen and its metabolites.

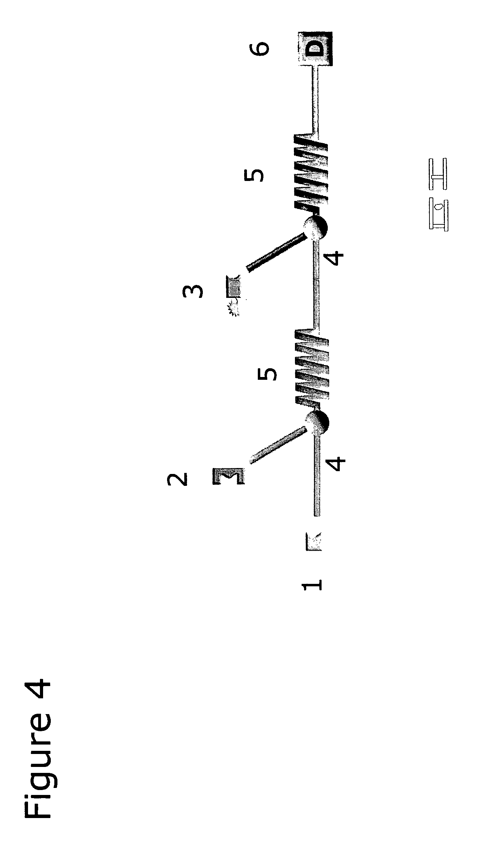

The biochemical assay to screen to tamoxifen is a homogeneous, continuous-flow binding assay (scheme, see Figure 4). The effluent of the LC column (FLOW 1) is mixed with a solution of the estrogen receptor in a mixing union. The solution of the estrogen receptor is pumped via a pumping devices containing a displacement loop and an HPLC pump. The reaction between the estrogen receptor and compounds eluting from the HPLC column takes place in an open-tubular reaction coil with a predefined volume. In a second stage of the biochemical assay a solution of a fluorescent estrogenic compound, coumestrol, is added. The reaction of coumestrol with estrogen receptor leads to a fluorescence enhancement effect which is reduced when active molecules elute from the HPLC column and block the binding site of the receptor. The reduction of fluorescence is measured by an HPLC fluorescence detector.

In detail, the bio-affinity detection system (Scheme; see Figure 4) consisted of two Agilent 1100 HPLC pumps for delivery of ER-α and coumestrol via two superloops. Two Agilent 1100 HPLC pumps were used to control the LC-gradient and two Agilent 1100 HPLC pumps were used directly after the HPLC-column to compensate for increasing organic modifier concentrations before delivery of the eluent to the bio-affinity detection system. ER-α and coumestrol solutions were delivered by means of so-called superloops (100 ml; made in house by the fine mechanical department) housed at 0 °C. The pressure limits of the pumps were set at a 20 bar higher pressure then the working pressure to prevent the superloops from exploding when a blockade occurs after the superloops. Injections were performed with a Gilson 234 autoinjector equipped with a Rheodyne six-port injection valve (injection loop, 75 µl or 450 µl). An Agilent 1100 series fluorescence detector (excitation wavelength 340 nm; emission wavelength 410 nm) was used for monitoring the fluorescence bio-affinity signal. An Agilent 1100 series UV detector monitored the HPLC eluent parallel to the bio-affinity signal at 280 nm. All hardware was controlled by Kiadis (Leiden, Netherlands) software.

For HPLC separations of the incubation mixtures a Phenomenex prodigy C18 (100×32) 5µ analytical column with a Phenomenex Security Guard column (C18-ODS (4mm L × 2 mm I.D.) AJO-4286 was used. A Shimadzu CTO-10AC column oven controlled the temperature of the reaction coil. The French pressure cell was from American Instrument Company (Cat. No. 4-3398A, Silver Spring Maryland, USA). A Branson Sonifier 250 with a model 102C microtip was used for sonification of cells.

All incubations were performed according to Lim *et al* (*vide supra*) with slight modifications: 0.65 mM of NADPH was used instead of NADP⁺. Instead of 2 ml microsomes per 10 ml of incubation mixture, 1 ml was used for the rat and pig microsomal incubations. For every incubation time 10 ml was used. Extraction of metabolites was as follows: first 5 ml of acetonitrile followed by two times 10 ml isopropylether. After evaporation with help of a rotary evaporator (45°C waterbath) reconstitution was done by dissolving the residue in 30% ethanol (500 µl). A second 500 µl of 100% ethanol dissolved all of the metabolites and mother compound. The two fractions were mixed together affording a total of 1 ml metabolite mixture, which was used directly for injections in the system.

Unless otherwise noted a 75 µl injection volume was used. The linear flow rate (250 µl/min) gradient started at 95 % aqueous phase (0.1% acetic acid in water) and 5% organic phase (99% methanol) for 2 minutes. Then a linear gradient increased the organic modifier concentration to 95 % in 24 minutes, which stayed constant for 5 minutes. Equilibration occurred in one minute. After the LC column a make-up flow was placed to compensate for increasing concentration of organic modifier. The total flow rate after the make-up process was 1000 µl/min with an organic modifier concentration of 24 %. The hold-up volume used for the delay of the reversed gradient of the two make-up pumps compared to the two LC gradient pumps was 150 µl. The aqueous and organic phases were identical to the ones used for the LC gradient. The total flow rate was split 1 to 9. The 900 µl/min part was split again. The UV detector was set at 280 nm for measuring a 750 µl/min flow rate after the second split. The remaining 150 µl/min was split to the MS. The 100 µl/min remaining from the first split was pumped into the bio-affinity detection system.

Mass spectrometric measurements were performed on a Finnigan DECA mass spectrometer, equipped with an ESI probe. During measurements, normal full scan MS or MS/MS data dependent scanning mode, was used in positive ion mode with a scan range of 50.0 to 600.0. The capillary temperature was set at 220 °C. The sheath gas and auxiliary gas flow rate equaled 70 and 30 arbitrary units, respectively. The DECA mass spectrometer was not tuned to a specific m/z value, instead, general settings were applied to cover a broad m/z range.

During MS/MS data dependent scanning the most intense ion present in the MS spectrum was automatically designated as parent mass for MS/MS measurements. Background ions, were specified up front and were automatically ignored as potential parent masses. The activation amplitude was set at 35.0%, whereas the q-value equaled 0.25. The isolation width of the parent mass was adjusted to 1.0 a.m.u.

### On-line Estrogen Receptor-α bioassay

The bio-affinity detection system in a homogeneous assay mode used was a modified ER-α assay of the one described by Oosterkamp *et al*. Analytical Chemistry 68 (1996), 1201-1206. Flow rate of the coumestrol solution was 50 µl/min. The ER-α solution was pumped through at a flow rate of 100 µl/min. The first reaction coil was a 0.25 mm I.D. Tefzel tubing (25 µl; O.D.=1/16") while the second reaction coil was a 0.25 mm I.D. Tefzel tubing (50 µl; O.D.=1/16"). The biochemical was thermostatted at 20°C. The ER-α solution was made by dissolving 40 µl/ml stock solution in buffer (10 mM monosodium hydrogen phosphate buffer (pH 7.4; adjusted with KOH) containing 150 mM NaCl). The ER-α (a kind gift of Dr Ruff; Laboratoire de Biologie et Génomique Structurales 1, IGBMC, rue Laurent Fries, BP 163, 67404 Illkirch, France) was grown according to Eiler et al (7), but without estradiol in the medium. The cells were pelleted at 4600 rpm for 60 min. and the pellet was subsequently dissolved in 10 mM monosodium hydrogen phosphate buffer (pH 7.4; adjusted with KOH) containing 150 mM NaCl (50 ml). The cells were again pelleted at 4000 rpm for 15 min. and the pellet was dissolved in 50 ml of the same buffer. This washing step was repeated two more times. After the last washing step, the pelleted cells were dissolved in 25 ml of the same buffer. Partial purification of the ER-α was performed by use of three French Press cycles (700 bar) and ultrasonic sound (microtip, 30% duty cycle output 7, 10 cycles) to break up the cells and finally ultracentrifugation (100.000 g) for one hour to obtain the soluble receptor in the supernatant. The soluble receptor was stored at -80°C.

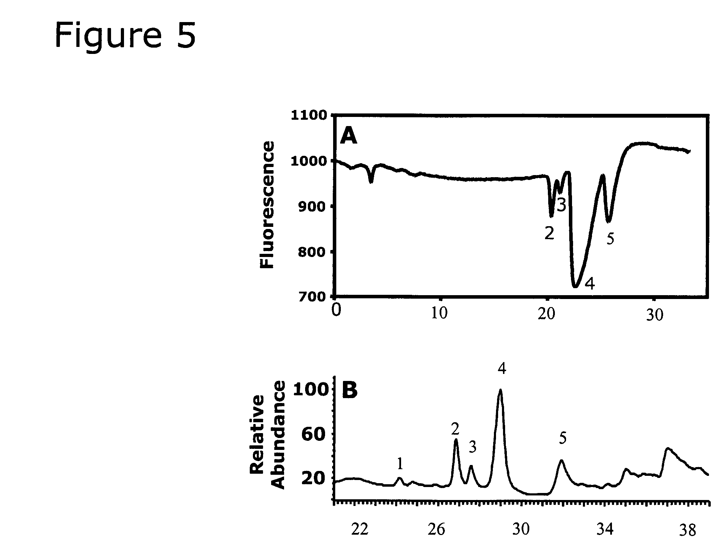

The configuration of Fig. 1A is used to screen the metabolic products produced after incubation of tamoxifen with liver microsomes. Figure 5 shows the information provided by this system. Molecules that generate positive biochemical responses in the bioassay are accurately correlated to the corresponding chemical data by performing retention time matching. As a first step in the data correlation process, the difference in dead volumes between analysis lines is determined by performing LC-BCD-MS analyses of reference compounds. In this way, the resulting difference in elution times between the biochemical assay and MS line is determined and used as a correction factor during the analysis of real samples. During these measurements the retention times of the biochemical responses are corrected after which the biological data is correlated to the chemical information recorded by MS. In a first step, the total ion current (TIC) is deconvoluted revealing a multitude of m/z values. These ions can potentially originate from co-eluting individual molecules, multiple charged species, dimers and fragments. In order to determine the ion(s) responsible for the biochemical response so called reconstructed ion currents (RIC) of each m/z trace are derived. The retention time of each mass at maximum peak height is compared with the corrected retention time of the biochemical response. Typically, the correlation is accurate within 0.2 min. In this way, mass traces eluting outside this time window are discarded, resulting in a dramatic decrease in mass traces. Co-eluting compounds are easily recognized by applying these selection criteria. Subsequently, the peak shapes of the remaining mass traces are compared with each other. Usually, molecules are partially fragmented in the mass spectrometer thus generating multiple mass traces. These traces however share almost exactly the same retention time and peak shape and consequently are easily recognized. The same is true for dimers and multiple charged molecules. In case mass traces are detected which indicate the co-elution of other molecules, the chromatographic conditions are adjusted to obtain higher resolution. In this way, the biochemical response can be correlated more accurately to the MS data.

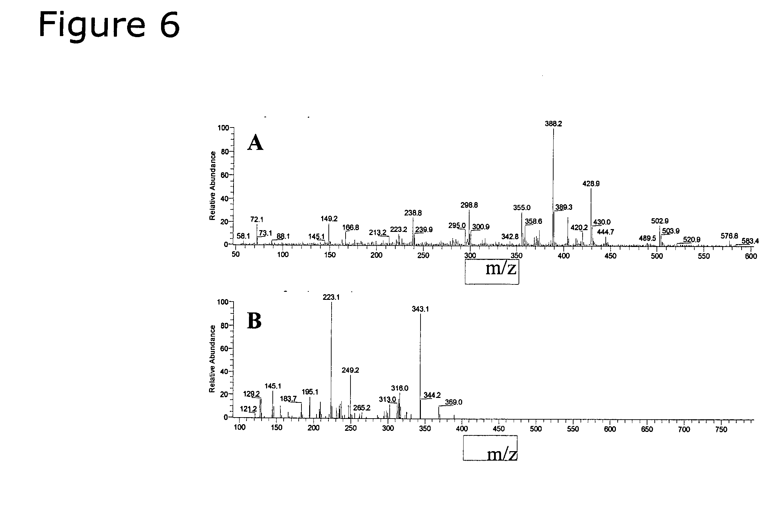

The described approach has been applied for the detection and identification of metabolites of tamoxifen. In the bioaffinity chromatogram (Figure 5A), four peaks are visible indicating the presence of multiple bioactive metabolites of tamoxifen. Subsequently the retention times of the metabolites were accurately determined at maximum peak height and corrected for the difference in dead volume between the biochemical and MS analysis line (Figure 5B). Subsequently, the observed biochemical responses were linked to the MS data. Peak number 1 in the bioaffinity chromatogram generates a MS spectrum in which several m/z traces are visible. After preparing the RIC's of each mass trace in the MS spectrum only one m/z value appeared to correspond well with the elution time and profile of the biochemical response. The retention times of the other m/z traces exceeded the 0.2 min correlation threshold window. The mass of the bioactive metabolite was determined to be 388.2 and was identified as hydroxytamoxifen based on the simultaneously recorded MS/MS spectrum (Figure 6). All other metabolites were identified in a similar manner. The results are summarized in Table 1.

**Table 1.**

| Summary detected bioactive metabolites of tamoxifen | | | | | |
|---|---|---|---|---|---|
| Peak # | Retention time bioassay (min) | Retention time MS (min) | Mass measured (Da) | Mass theoretical (Da) | Identity compound |
| 1. | -^{a} | 24.2 | 404.2 | 404.5 | dihydroxytamoxifen |
| 2. | 20.3 | 26.8 | 388.2 | 388.5 | hydroxytamoxifen |
| 3. | 21.3 | 27.6 | 388.2 | 388.5 | hydroxytamoxifen |
| 4. | 22.5 | 29.0 | 372.2 | 372.5 | tamoxifen |
| 5. | 25.3 | 31.9 | 388.2 | 388.5 | tamoxifen-N-oxide |

| | | | | | |
|---|---|---|---|---|---|
| ^{a} Presence only observed in TIC chromatogram. Concentration of compound to low to generate response in the biochemical assay. | | | | | |

## Claims

1. A method for identifying active and/or inactive metabolites of at least one active component, comprising the steps of:
a) providing a mixture starting from the at least one active component, by subjecting said at least one active component to a metabolising step giving active and/or inactive metabolites;
b) fractionating said mixture;
c) dividing the fractionated mixture in at least two fractionation streams;
d) subjecting one of the fractionation streams to a biochemical detection;
e) subjecting in parallel another fractionating stream to a chemical characterization step;
f) determining on the basis of the parallel results provided by steps d) and e) whether the metabolites present are active.

2. The method of claim 1, wherein the chemical characterization step is a mass spectrometry step.

3. The method of claim 1 or 2 wherein the biochemical detection comprises fluorescence spectrometry.

4. The method of any one of claims 1-3, wherein the fractionated mixture is divided in three fractionation streams, wherein in the third stream a chemical detector different from the detectors in the first two streams is present.

5. The method of claim 4, wherein the chemical detector in the third stream is a UV-VIS spectrometer.

6. The method of any one of the preceding claims wherein the mixture provided in step a results from an incubation using at least one enzyme.

7. The method of claim 6, said enzyme comprising cytochrome P450.

8. The method of any one of the preceding claims, wherein the fractionation step is a liquid chromatography step.

9. A method according to any of the preceding claims, wherein the biochemical detection is an on-line continuous flow biochemical assay.

10. A method according to any of the preceding claims, wherein furthermore a stream is lead to a diode-array detector, and subsequently to a fluorescence detector.

11. A method according to claim 10, wherein the detector is an evaporative light scattering detector.

12. A method for the simultaneous measurement of half-lives of active and inactive metabolites, the determination of time dependent concentration, the determination of inhibition of metabolic enzymes, using the metabolic profiling method of one of the preceding claims.

13. Active metabolite obtainable by the method of any one of the claims 1-12.

14. The use of a product obtainable by any of the preceding claims for the manufacture of a medicament.
